# NOUVEAU FASCICULE DE BREVET EUROPEEN

(11) **EP 1 589 926 B2**
(45) Date de publication et mention de la décision concernant l'opposition: **25.07.2018**
(45) Mention de la délivrance du brevet: 01.10.2008
(21) Numéro de dépôt: 03816148.5
(22) Date de dépôt: 24.12.2003
(51) Int. Cl.: A61M 5/178, A61M 5/00, A61J 1/20

(54) **DISPOSITIF ET PROCÉDÉ DE PRÉPARATION EXTEMPORANÉE D'UNE QUANTITÉ INDIVIDUELLE DE FLUIDE STÉRILE**
VORRICHTUNG UND VERFAHREN ZUR EXTEMPORIERTEN PRÄPARATION EINER STERILEN FLÜSSIGEN DOSIS
DEVICE AND METHOD FOR THE EXTEMPORANEOUS PREPARATION OF AN INDIVIDUAL QUANTITY OF STERILE FLUID

(30) Priorité: 05.02.2003 FR 0301334
(43) Date de publication de la demande: 02.11.2005
(73) Titulaire: Arcadophta, 31100 Toulouse (FR)
(72) Inventeur: REBOUL, Gérard, F-31400 Toulouse (FR)
(74) Mandataire: Lassiaille, Christian Michel
(86) Numéro de dépôt international: PCT/FR2003/003890
(87) Numéro de publication internationale: WO 2004/078094

(56) Documents cités:
- WO-A1-00/10423
- WO-A1-00/24649
- WO-A1-82/00780
- US-A- 3 853 157
- US-A- 5 569 209
- US-A- 6 073 759
- US-B1- 6 494 314
- US-B1- 6 599 280

## Description

L'Invention concerne un dispositif et un procédé de préparation extemporanée d'une quantité individuelle de fluide de traitement stérile pouvant être compressible -notamment un gaz de traitement- en vue de son administration à un patient (humain ou animal).

Certaines applications thérapeutiques ou chirurgicales exigent l'administration d'un fluide, dit fluide de traitement stérile, et compressible notamment un gaz, par exemple de l'air ou un gaz fluorocarboné (CF₄, C₂F₆, C₃F₈, ...), SF₆, N₂, CO₂, ... Par exemple certains actes de chirurgie ophtalmologiques nécessitent l'administration topique d'un tel gaz de traitement neutre parfaitement stérile dans la cavité oculaire.

Le problème général qui se pose donc dans ce contexte est celui de mettre à la disposition du praticien une quantité individuelle (c'est-à-dire destinée à un patient unique) de ce fluide à l'état parfaitement stérile, et dans des conditions appropriées à l'administration envisagée.

Jusqu'à maintenant, dans la pratique, les quantités individuelles de gaz de traitement sont préparées par remplissage d'une seringue d'administration à partir d'une bouteille de gaz de grand volume sous forte pression et via un détendeur et un dispositif de stérilisation par filtration. Néanmoins, ces bouteilles de grandes tailles et le gaz qu'elles contiennent ne peuvent pas être stérilisés ni maintenus à l'état stérile dans des conditions satisfaisantes. Le passage de la seringue depuis le site non stérile de remplissage à la bouteille jusqu'au site stérile d'utilisation (par exemple bloc chirurgical) exige de nombreuses manipulations et précautions, entraîne des risques de contaminations, et ne permet pas de procurer une « traçabilité » ad hoc de chaque quantité individuelle de fluide de traitement administrée à chaque patient.

US-3 853 157 décrit un appareil pour remplir des seringues en composition liquide à partir d'une cartouche rigide contenant un volume de liquide correspondant à plusieurs doses individuelles, et dotée d'une valve à bouton d'actionnement manuel. La valve peut être du type doseuse pour faciliter le remplissage rapide d'un grand nombre de seringues. Ce dispositif ne permet pas de garantir la stérilité de la composition liquide introduite dans la seringue, qui peut être contaminée notamment au contact de l'orifice de sortie de la valve de la cartouche, susceptible d'être infecté. Il n'est pas non plus compatible avec la distribution d'une composition compressible (gazeuse ou contenant un gaz) qui rendrait extrêmement délicat, sinon impossible, le dosage précis, par simple visualisation du déplacement du piston, du volume introduit dans la seringue.

US 6073759 décrit un dispositif qui comprend une seringue reliée à un réservoir de gaz pressurisé. La pression dans la réservoir est suffisante pour remplir la seringue. Le réservoir comprend une valve opérée manuellement avec un bouton. Un filtre en HEPA est interposé entre le réservoir et la seringue pour retenir les particules. La seringue est incorporée dans un conteneur de plus grande taille, ce conteneur étanche au gaz, contenant lui-même le même gaz sous pression. Ce document indique que, dans le mode de réalisation de la figure 7, le bouton est actionné pour délivrer le gaz à partir du réservoir pour remplir la seringue et que, typiquement, le réservoir contient une quantité suffisante de gaz pour nettoyer la seringue afin d'éliminer tout contaminant pouvant être présent.

L'Invention vise donc à pallier ces inconvénients en proposant un dispositif permettant la préparation extemporanée, sur le site d'utilisation, d'une quantité individuelle de fluide de traitement stérile pouvant être compressible, simple et fiable d'utilisation, évitant toute fuite de fluide lors de l'assemblage et de la préparation -notamment totalement hermétique lors du remplissage de la seringue-, et tout risque de rupture de l'asepsie, et pouvant être lui-même complètement stérilisé -notamment à l'oxyde d'éthylène- sans risque -notamment sans risque de mélange ou de réaction du fluide de traitement avec l'oxyde d'éthylène-.

L'invention vise en particulier à proposer un tel dispositif qui peut être utilisé et qui est même particulièrement adapté et avantageux, dans le cas où le fluide de traitement est compressible, c'est-à-dire constitué d'un gaz ou comprenant un gaz.

L'invention vise aussi à proposer un tel dispositif qui permet d'assurer le suivi (« traçabilité ») des doses de fluide administrées.

L'invention vise également à proposer un tel dispositif qui soit de fabrication simple et peu coûteuse, et ne comprenne qu'un nombre réduit de pièces.

Pour ce faire, l'invention concerne un dispositif conforme à la revendication 1.

Autrement dit, le cylindre de la seringue est adapté pour pouvoir contenir toute la quantité de fluide de traitement (quantité individuelle) pouvant être libérée par la cartouche à la pression atmosphérique.

Le fait d'utiliser une cartouche rigide permet notamment en pratique de réaliser une stérilisation parfaite de l'ensemble du dispositif. En effet, une telle cartouche rigide peut être notamment réalisée en matière métallique formant une barrière parfaite au fluide de stérilisation tel que l'oxyde d'éthylène. En outre, une telle cartouche rigide peut renfermer le fluide de traitement sans aucun risque de contamination de ce fluide de traitement

Par ailleurs, en prévoyant que la cartouche rigide pressurisée ne contient qu'une quantité individuelle de fluide de traitement, c'est-à-dire une dose destinée à une administration unique, l'invention va exactement à l'encontre de l'art antérieur où l'on cherche toujours au contraire à renfermer une quantité maximum de fluide de traitement dans un minimum de volume. Mais, cela permet de faciliter l'opération de remplissage de la seringue, y compris dans le cas d'un gaz de traitement, en évitant à la fois l'expulsion intempestive du piston hors du cylindre, l'introduction d'une quantité insuffisante, et l'emploi d'une valve doseuse, généralement inefficace ou imprécise dans le cas d'un gaz, et en tout cas très onéreuse. Ainsi, le remplissage de la seringue s'effectue automatiquement sans nécessiter aucun contrôle visuel du dosage de la quantité introduite dans la seringue de la part de l'utilisateur y compris dans le cas où le fluide de traitement est compressible. En pratique, il suffit de prévoir que le volume maximum du cylindre de la seringue (piston en position extrême éloigné de l'extrémité de distribution de la seringue) soit supérieur à la quantité individuelle de fluide de traitement destinée à une administration unique pouvant être libérée par la cartouche, qui peut néanmoins être elle-même supérieure ou égale à la quantité posologique requise pour le traitement du patient (l'utilisateur (praticien) pouvant ensuite aisément refaire sortir le surplus de fluide de traitement hors du cylindre en repoussant le piston avant l'administration).

Par ailleurs, le dispositif selon l'invention étant à usage unique et doté d'un dispositif de stérilisation par filtration du fluide de traitement pénétrant dans la seringue elle-même préalablement stérilisée, le caractère stérile de la quantité de fluide de traitement contenu dans la seringue est garanti. En particulier, on minimise les risques de contamination -notamment au niveau de la valve de sortie de la cartouche- par des micro-organismes pathogènes pouvant résulter d'un usage multiple du même réservoir de fluide. Et même en cas d'infection des parties extérieures de la valve de sortie, le fluide est stérilisé par le dispositif de stérilisation avant d'entrer dans la seringue. En outre, avec des cartouches à quantité individuelle et usage unique, il est possible de réaliser le suivi des quantités de fluide administrées (« traçabilité »).

Avantageusement et selon l'invention, la valve de sortie est du type rappelée élastiquement axialement vers l'extérieur en position d'obturation et pouvant être repoussée axialement vers l'intérieur de la cartouche en vue de son ouverture, et le dispositif de raccordement/stérilisation est adapté pour réaliser un assemblage axial de la seringue, de ce dispositif de raccordement/stérilisation, et de la valve de la cartouche, et pour permettre le remplissage de la seringue, sans fuite de fluide de traitement, par simple rapprochement axial de la seringue et de la cartouche. Dès lors, la manipulation du dispositif selon l'invention aussi bien pour l'assemblage que pour le remplissage de la seringue est particulièrement simple et ergonomique. Elle ne nécessite que deux points d'appui (contrairement au dispositif de US-3 853 157 qui nécessite trois points d'appui).

En outre, avantageusement et selon l'invention, le dispositif de raccordement/stérilisation comprend une portion de raccordement à la valve de la cartouche, et cette portion de raccordement et cette valve sont adaptées pour que :
- dans une première course de rapprochement axial relatif, un raccord hermétique puisse être réalisé entre elles, sans actionnement de la valve de la cartouche qui reste fermée,
- dans une deuxième course de rapprochement axial relatif ultérieur au-delà de la première course, la valve soit ouverte.

Un tel dispositif permet en particulier d'éviter tout risque de fuite de fluide au moment même de l'assemblage et du raccordement, puisque la valve reste complètement fermée tant que le raccordement n'est pas complètement hermétique.

Avantageusement et selon l'invention, la valve de la cartouche est une valve femelle dotée d'un orifice de distribution, d'un joint périphérique d'étanchéité, et d'une cavité d'actionnement adaptée pour recevoir une extrémité d'un ajutage bi-fonctionnel d'actionnement de la valve et d'éjection du fluide de traitement, et ladite portion de raccordement du dispositif de raccordement/stérilisation forme un ajutage bi-fonctionnel compatible avec la valve femelle de la cartouche. Un tel mode de réalisation est particulièrement simple et avantageux, peu coûteux, commode et fiable à l'utilisation.

Avantageusement et selon l'invention, dans le mode de réalisation où la valve est une valve femelle, le dispositif de raccordement/stérilisation comprend un dispositif de liaison hermétique amont présentant, d'un côté, ledit ajutage bi-fonctionnel compatible avec la valve femelle de la cartouche, et de l'autre côté, une connexion compatible avec l'entrée du dispositif de stérilisation par filtration.

Avantageusement et selon l'invention, ladite connexion du dispositif de liaison hermétique amont est une liaison tronconique -notamment normalisée (NF EN 1615) de type Luer®-, par exemple femelle, conjuguée d'une liaison tronconique -notamment normalisée de type Luer®- par exemple mâle, formée par l'entrée du dispositif de stérilisation par filtration.

Avantageusement et selon l'invention, la sortie du dispositif de stérilisation par filtration est adaptée pour pouvoir être connectée directement -sans raccord intermédiaire- à l'extrémité de distribution de la seringue d'administration.

Avantageusement et selon l'invention, la sortie du dispositif de stérilisation par filtration est une liaison tronconique femelle -notamment normalisée de type Luer®- conjuguée d'une liaison tronconique mâle -notamment normalisée de type Luer®- formée par l'extrémité de distribution de la seringue d'administration.

En variante, il est néanmoins possible de prévoir un dispositif de liaison aval destiné à être interposé entre le dispositif de stérilisation par filtration et l'extrémité de distribution de la seringue d'administration. Un tel dispositif de liaison aval peut par exemple être doté d'un robinet d'arrêt permettant notamment d'éviter toute entrée ou sortie de fluide hors de la seringue lors de la mise en place de l'aiguille d'administration sur la seringue.

Par ailleurs, avantageusement et selon l'invention, la cartouche comprend une poche interne souple renfermant ladite quantité individuelle de fluide de traitement sous pression raccordée à la valve de sortie, et à l'extérieur de la poche (mais à l'intérieur de la cartouche), une quantité de gaz pousseur (à l'état gazeux) adaptée à la mise sous pression du fluide de traitement contenu dans la poche. Avantageusement et selon l'invention, le gaz pousseur est de même nature que le fluide de traitement, notamment identique au fluide de traitement lorsque ce dernier est un gaz de traitement. Ainsi, le gaz pousseur ne constitue pas en lui-même un risque de toxicité ou de contamination pour le patient.

Avantageusement et selon l'invention, la pression du fluide de traitement dans la cartouche est faiblement supérieure à la pression atmosphérique, de façon à éviter tout accident et toute fuite intempestive lors de l'assemblage et du remplissage de la seringue. Avantageusement et selon l'invention, la quantité de gaz pousseur est adaptée pour maintenir dans la poche (lorsque celle-ci contient la quantité individuelle de fluide de traitement) une surpression (par rapport à la pression atmosphérique) de fluide de traitement inférieure à 2000 hPa -notamment de l'ordre de 600 à 1800 hPa-. De la sorte, il n'est pas nécessaire de prévoir un détendeur ni un manomètre, ni un robinet d'arrêt pour le remplissage de la seringue à partir de la cartouche.

La pression initiale du gaz pousseur à l'état gazeux est adaptée pour refouler toute quantité individuelle de fluide de traitement. En pratique, cette pression initiale peut être adaptée pour qu'en fin de distribution de la quantité individuelle de fluide de traitement, la pression résiduelle du gaz pousseur corresponde à la pression atmosphérique ou soit peu supérieure à la pression atmosphérique, par exemple de l'ordre de 1050 hPa. La faible surpression correspondante (de l'ordre de 20 à 70 hPa) s'avère être en pratique largement suffisante pour expulser toute la quantité individuelle de fluide de traitement contenue dans la poche, notamment dans le cas d'un gaz de traitement. Cette faible valeur de surpression (résultant de l'emploi d'un gaz pousseur à l'état gazeux) pour l'expulsion du fluide de traitement est aussi importante pour le choix, le dimensionnement et la garantie de l'efficacité du dispositif de stérilisation par filtration. En effet, les filtres de stérilisation ne résistent en général pas aux pressions importantes, telles que celles qui seraient imparties (typiquement 6 à 20 fois la pression atmosphérique) par l'emploi d'un gaz pousseur liquéfié choisi parmi les gaz compatibles avec une stérilisation à l'oxyde d'éthylène.

Par ailleurs, avantageusement et selon l'invention, la cartouche comprend un volume de fluide de traitement compris entre 10 ml et 100 ml -notamment de l'ordre de 40 ml-, inférieur à la capacité totale de la seringue.

En outre, avantageusement et selon l'invention, la cartouche comprend un opercule de fermeture hermétique de l'orifice de la valve femelle adapté pour empêcher toute pénétration de fluide de stérilisation dans la valve et la cartouche, et pour pouvoir être brisé par introduction de l'ajutage bi-fonctionnel dans l'orifice de la valve. Cet opercule, qui comprend par exemple une pellicule d'aluminium, permet d'éviter tout contact du fluide de traitement et/ou de la valve de la cartouche avec le fluide de stérilisation -notamment l'oxyde d'éthylène-, qui est toxique.

Avantageusement, le dispositif selon l'invention comprend en outre une aiguille stérile apte à être raccordée à l'extrémité de distribution de la seringue pour l'administration du fluide de traitement au patient ; et une étiquette -notamment sous forme de bracelet- destinée à être associée au patient, pouvant porter une référence identifiant la cartouche de fluide de traitement utilisée. Ce bracelet permet de signaler au personnel soignant le fait que le patient a été traité par le fluide de traitement, de sorte que des précautions appropriées peuvent être prises pour les soins et traitements ultérieurs. Cela est particulièrement avantageux et important dans le cas d'un gaz de traitement.

Le dispositif selon l'invention comprend en outre une enveloppe externe d'emballage stérile renfermant l'intégralité des éléments constitutifs du dispositif à l'état stérile. Il se présente ainsi en un paquet unique emballé stérile prêt à l'emploi sur le site d'administration.

Avantageusement et selon l'invention, la seringue et le dispositif de stérilisation se présentent préalablement assemblés à l'état stérile dans l'enveloppe. En particulier, la seringue et le dispositif de raccordement/stérilisation sont préalablement assemblés et se présentent stériles et assemblés dans l'enveloppe. De la sorte, on garantit que la cavité interne de la seringue recevant le fluide de traitement ne puisse jamais venir être contaminée, même à l'ouverture de l'enveloppe, grâce au dispositif de stérilisation qui empêche toute pénétration de germe dans cette cavité. En outre, même si la sortie de la valve de la cartouche, ou l'opercule qu'elle porte est infectée, le fluide de traitement pénétrant dans la seringue est stérilisé.

L'invention s'étend à un procédé de préparation extemporanée d'une quantité individuelle de fluide de traitement stérile pouvant être compressible -notamment un gaz de traitement- en vue de son administration à un patient, à l'aide d'un dispositif selon l'invention.

Avantageusement et selon l'invention, on assemble axialement la seringue, le dispositif de raccordement/stérilisation et la valve de la cartouche, de façon à réaliser entre eux une communication isolée hermétiquement de l'extérieur, puis on rapproche axialement la seringue et la cartouche de façon à ouvrir la valve et à introduire dans la seringue une dose prédéterminée de fluide de traitement stérile, par équilibrage des pressions. Le fluide de traitement peut ensuite être utilisé pur ou dilué. Dans ce dernier cas, lorsque le fluide de traitement est un gaz de traitement, on dilue le fluide de traitement dans la seringue en :
- repoussant le piston pour éjecter le surplus de fluide de traitement hors de la seringue,
- tirant le piston pour introduire dans la seringue une quantité d'air atmosphérique de dilution via le dispositif de stérilisation par filtration.

Avantageusement et selon l'invention, la seringue et le dispositif de stérilisation -notamment le dispositif de raccordement/stérilisation- étant préalablement assemblés, on réalise l'assemblage axial uniquement en reliant l'entrée du dispositif de stérilisation -notamment l'ajutage bi-fonctionnel- à la valve de la cartouche.

Avantageusement et selon l'invention, on dissocie ensuite (après préparation de la quantité de fluide de traitement à administrer dans la seringue) le dispositif de stérilisation par filtration de la seringue à laquelle on raccorde une aiguille d'administration. On peut ensuite utiliser la seringue avec l'aiguille pour l'administration du fluide de traitement au patient.

Le dispositif et le procédé selon l'invention sont particulièrement simples, fiables, et garantissent une grande sécurité d'utilisation, sans risque de fuite, d'accident ni de contamination, y compris dans le cas d'un fluide de traitement compressible, pour lequel il n'existe, à ce jour, aucune solution satisfaisante (les problèmes résolus par l'invention dans ce cas n'ayant d'ailleurs jamais été posés). Ils permettent aussi de garantir le suivi des doses de fluide administrées (« traçabilité »).

L'invention concerne aussi un dispositif et un procédé caractérisés en combinaison par tout ou parties des caractéristiques mentionnées ci-dessus ou ci-après.

D'autres buts, caractéristiques et avantages de l'invention apparaîtront à la lecture de la description suivante d'un de ses modes de réalisation donné à titre d'exemple non limitatif, et qui se réfère aux figures annexées dans lesquelles :
- la figure 1 est un schéma en perspective illustrant un exemple de dispositif selon l'invention,
- la figure 2 est une vue en coupe d'un exemple de réalisation d'une valve de cartouche de fluide de traitement d'un dispositif selon l'invention avant utilisation,
- la figure 3 est une vue similaire à la figure 2 illustrant la valve au cours du remplissage de la seringue,
- les figures 4 à 8 sont des vues schématiques en coupe illustrant respectivement différentes étapes d'un procédé selon l'invention.

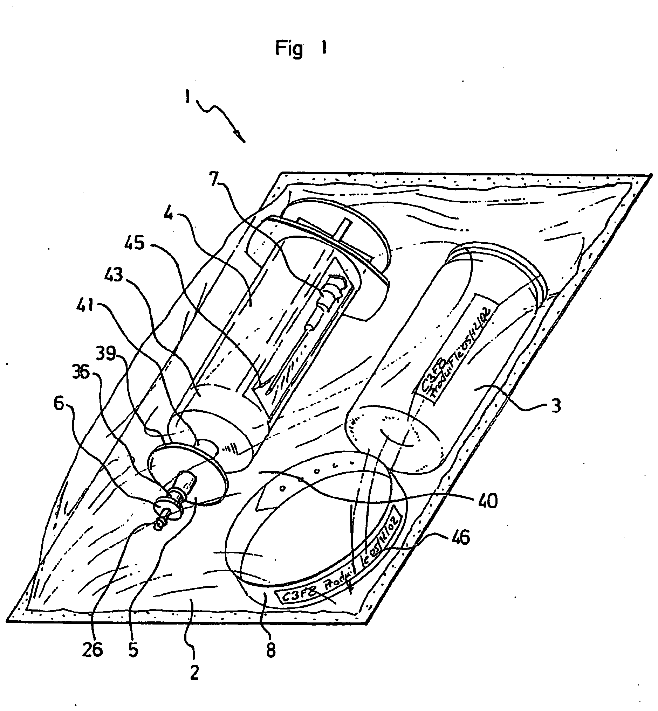

Le dispositif 1 selon l'invention représenté figure 1 se présente à l'état conditionné stérile, prêt à l'emploi, et comprend :
- une enveloppe externe 2 d'emballage stérile définissant une enceinte close hermétique renfermant les différents éléments constitutifs du dispositif 1, à savoir :
   - une cartouche rigide 3 faisant office de récipient contenant une quantité individuelle de fluide de traitement -notamment un gaz de traitement-sous pression,
   - une seringue d'administration 4,
   - un dispositif 5 de stérilisation par filtration,
   - un dispositif 6 de liaison hermétique amont destiné à être interposé entre le dispositif 5 de stérilisation par filtration et la cartouche 3,
   - une aiguille d'administration 7,
   - un bracelet 8 destiné à être porté par le patient.

Tous ces éléments constitutifs sont de type jetable à usage unique.

La cartouche 3 comprend un boîtier externe 9 métallique -notamment en aluminium ou en fer blanc- rigide définissant une ouverture d'extrémité refermée par une valve 10 de sortie portant une poche 11, par exemple du type de celle commercialisée sous la marque EPSPRAY SYSTEM® par la société EP SPRAT SYSTEM SA (Neuchâtel, Suisse), s'étendant à l'intérieur du boîtier 9 et associée avec son enceinte interne en communication de fluide avec la valve 10.

La valve 10 comprend une paroi métallique rigide d'obturation 12 fixée à l'extrémité du boîtier 9 de façon hermétique par l'intermédiaire d'un joint d'étanchéité 13, et présentant un orifice circulaire central 14 de distribution. Un manchon 15 rigide est fixé sous l'orifice 14 par sertissage de ce manchon 15 par une portion rétrécie de la paroi 12 serrée contre un épaulement externe 16 de sertissage du manchon 15. Un joint d'étanchéité périphérique 17 est interposé entre l'extrémité du manchon 15 et la portion de paroi 12 définissant l'orifice 14 de distribution. Ce joint 17 présente une lumière circulaire 25 sous l'orifice 14.

Le manchon 15 définit à son extrémité supérieure un alésage 18 de plus grand diamètre que l'orifice 14 et que le diamètre interne de la lumière circulaire 25 définie par le joint d'étanchéité 17 qui est lui-même légèrement plus petit que celui de l'orifice de distribution 14. Cet alésage 18 contient une soupape 19 présentant un bord 20 d'obturation supérieur en forme de couronne circulaire adapté pour venir au contact de la paroi inférieure du joint 17 d'étanchéité s'étendant en regard de l'alésage 18. La soupape 19 est rappelée élastiquement axialement à l'encontre du joint 17 d'étanchéité par un ressort de compression 21 disposé à l'intérieure de l'alésage 18, et prenant appui d'un côté contre un épaulement 22 interne de l'alésage 18 et, de l'autre côté, contre un épaulement 23 de la paroi externe de la soupape 19.

La paroi externe de la soupape 19 présente un encombrement radial inférieur à celui de l'alésage 18 au niveau du bord d'obturation 20, de sorte qu'un jeu est ménagé radialement entre le bord d'obturation 20 et l'alésage 18. Ce jeu permet le passage du fluide autour de la soupape 19 puis entre le joint 17 et le bord d'obturation 20 lorsque la soupape 19 est repoussée vers le bas à l'encontre du ressort 21.

La soupape 19 comprend en outre une cavité d'actionnement 24 en forme de tronc de pyramide adaptée pour recevoir l'extrémité axiale 34 symétrique de révolution d'un ajutage 26 rigide bi-fonctionnel d'éjection du fluide de traitement et d'actionnement de la valve 10. Cet ajutage 26 est formé d'une portion tubulaire creuse symétrique de révolution. Il est bi-fonctionnel en ce sens qu'il peut exercer une première fonction consistant à actionner la soupape 19 et la valve 10 pour l'ouvrir, et une deuxième fonction consistant à réaliser un passage d'éjection du fluide de traitement hors de la valve 10 et de la cartouche 3. La cavité 24 d'actionnement présente une ouverture 27 d'extrémité supérieure dont les dimensions radiales sont légèrement supérieures à celles de la lumière 25 du joint d'étanchéité 17 et à celles de l'orifice 14 de distribution. La cavité 24 d'actionnement est refermée à son extrémité inférieure par un fond 28 de la soupape 19, de sorte qu'aucun passage de fluide de traitement n'est possible à travers la soupape 19 lorsque cette dernière est repoussée par le ressort 21 avec son bord d'obturation 20 contre le joint 17 d'étanchéité. La cavité 24 pyramidale est de section transversale polygonale, par exemple triangulaire, de sorte qu'un passage de fluide de traitement est nécessairement ménagé entre la paroi interne de cette cavité 24 et l'extrémité axiale 34 symétrique de révolution de l'ajutage 26 insérée dans cette cavité 24.

La poche 11 est fixée de façon hermétique, par exemple par collage, contre la paroi externe du manchon 15 dont l'alésage 18 débouche à l'intérieur de la poche 11. Cette poche 11 définit donc, d'une part, une enceinte interne 29 qui contient le fluide de traitement et est en communication avec l'alésage 18 jusqu'à la soupape 19, et, d'autre part, entre cette poche 11 et la paroi rigide externe du boîtier 9, une enceinte intermédiaire 30 renfermant une quantité appropriée de gaz pousseur pressurisé à l'état gazeux. Ce gaz pousseur est de préférence un gaz physiologiquement acceptable, compatible avec le fluide de traitement et avec le procédé de stérilisation, notamment avec l'oxyde d'éthylène. De la sorte, on évite, d'une part, toute complication en cas de diffusion accidentelle du gaz pousseur dans le fluide de traitement, et, d'autre part, tout risque d'accident au cours de l'étape de stérilisation à l'oxyde d'éthylène.

La quantité de gaz pousseur contenue dans l'enceinte intermédiaire 30 est adaptée pour pressuriser le fluide de traitement dans l'enceinte 29 de la poche 11 à une pression relative appropriée. Avantageusement et selon l'invention, la quantité de gaz pousseur est adaptée pour maintenir dans la poche 11 une surpression de fluide de traitement (par rapport à la pression atmosphérique) inférieure à 2 000 hPa -notamment de l'ordre de 600 à 1 800 hPa-. Cette surpression est de valeur relativement faible mais suffisante pour le remplissage spontané de la seringue (le piston étant mû par la pression du fluide de traitement expulsé de la valve) comme expliqué ci-après, sans détendeur, ni robinet d'arrêt. La poche 11 est par ailleurs d'un volume correspondant à celui de la quantité individuelle de fluide de traitement devant être délivrée par la cartouche 3, inférieur ou égal au volume maximum du cylindre de la seringue 4. En pratique, ce volume de fluide de traitement est typiquement compris entre 10ml et 100ml -notamment de l'ordre de 40ml-.

L'invention est plus particulièrement avantageusement applicable au cas où le fluide de traitement est un gaz de traitement. Ce gaz de traitement peut être notamment choisi parmi les gaz fluorocarbonés (CF₄, C₂F₆, C₃F₈ ...), SF₆, N₂, C0₂ ou de l'air. Avantageusement, dans un dispositif selon l'invention, le gaz pousseur est de même nature que le fluide de traitement. En particulier, l'inventeur a constaté que la plupart des gaz de traitement peuvent en pratique faire office de gaz pousseurs. Tel est en particulier le cas des gaz fluorocarbonés tel que CF₄, C₂F₆, C₃F₈ ..

L'orifice 14 de distribution de la paroi 12 d'obturation de la valve 10 est avantageusement refermé de façon hermétique avant utilisation par un opercule 31 de fermeture hermétique de cet orifice 14. Cet opercule 31 est collé hermétiquement à l'extérieur de la paroi 12 et est adapté pour empêcher toute pénétration de fluide de stérilisation dans la valve 10, notamment dans la cavité 24 et dans l'alésage 18, c'est-à-dire dans la poche 11. Pour ce faire, dans le cas notamment où on utilise l'oxyde d'éthylène à titre de gaz de stérilisation, l'opercule 31 comprend au moins une feuille d'aluminium faisant office de barrière hermétique à l'oxyde d'éthylène. Cet opercule 31 est néanmoins suffisamment fin pour pouvoir être brisé par introduction de l'ajutage bi-fonctionnel 26 dans l'orifice 14 de la valve 10 en vue du remplissage d'une seringue. L'opercule 31 est en matériau non élastique de façon à permettre une rupture facile de cet opercule 31 par l'ajutage 26. Il doit néanmoins être suffisamment épais pour offrir une résistance imposant l'exercice d'un effort axial au-delà d'une valeur seuil pour entraîner sa rupture. De la sorte, on évite toute rupture involontaire de l'opercule 31, et donc tout actionnement intempestif de la valve 10.

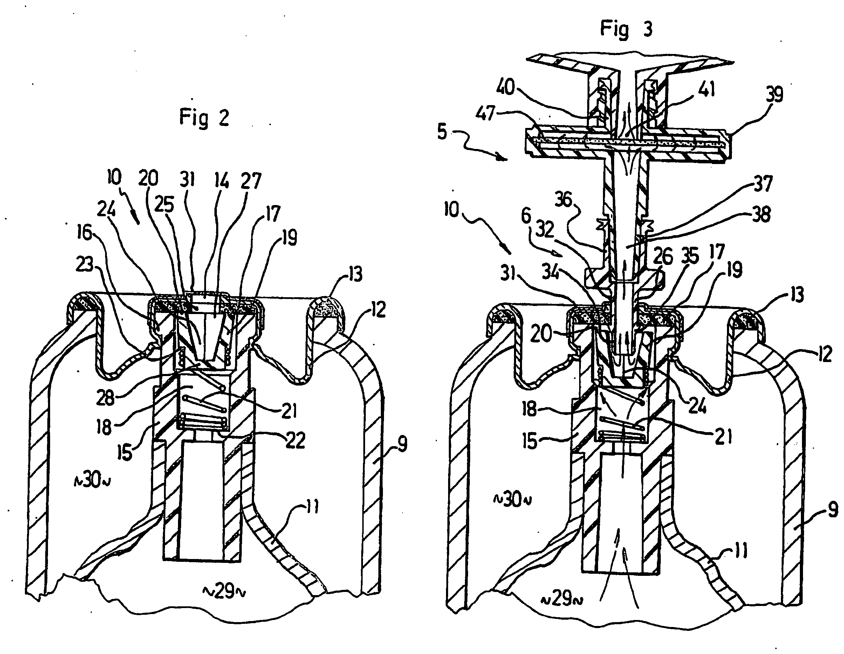

Avant utilisation, la cartouche 3 se présente comme représenté figure 2 et contient donc une quantité individuelle de fluide de traitement à l'intérieur de l'enceinte 29 de la poche 11 sous une pression relative de l'ordre de 600 à 1 800 hPa, maintenue par une pression relative identique de gaz pousseur dans l'enceinte intermédiaire 30.

Le dispositif 6 de liaison hermétique amont est un raccord en matière synthétique pouvant être stérilisé formant d'un côté ledit ajutage 26 bi-fonctionnel compatible avec la valve 10 femelle de la cartouche 3, et de l'autre côté, une connexion tronconique 36 -notamment normalisée de type Luer®- compatible avec l'entrée de fluide 33 du dispositif 5 de stérilisation par filtration. Ce raccord 6 est creux de façon à former un passage de fluide axial à travers l'ajutage 26 et sur toute sa longueur.

L'ajutage 26 bi-fonctionnel formé par ce raccord 6 doit présenter des dimensions compatibles avec celles de la valve 10, c'est-à-dire avec celles de l'orifice 14 de distribution de la lumière 25, du joint d'étanchéité 17, et de la cavité 24 d'actionnement de la soupape 19. Il doit être suffisamment rigide pour permettre de briser l'opercule 31 et repousser la soupape 19 à l'encontre du ressort 21 axialement.

Comme on le voit figure 3, l'ajutage 26 présente une paroi externe 32 cylindrique de révolution de diamètre légèrement supérieur à celui de la lumière 25 circulaire du joint d'étanchéité 17, de sorte que lorsque l'on introduit cet ajutage 26 dans l'orifice 14, le joint d'étanchéité 17 vient en contact d'une portion de cette paroi externe 32 en assurant l'étanchéité au fluide de traitement, notamment au gaz de traitement. L'extrémité axiale 34 de l'ajutage 26 est de préférence chanfreinée pour faciliter la rupture de l'opercule 31 et peut être d'un diamètre légèrement inférieur à la portion de paroi externe 32 venant au contact du joint 17. La paroi externe 32 de l'ajutage 26 peut comprendre par ailleurs une surépaisseur 35 entre l'extrémité axiale 34 et la portion cylindrique de cette paroi 32 venant au contact du joint 17. Cette surépaisseur 35 est de préférence de forme tronconique comme représenté se rétrécissant vers l'extrémité axiale 34, et présente un épaulement anti-retour de liaison avec la paroi externe 32, de sorte qu'elle peut être introduite en force, par déformation élastique, à travers l'orifice 14 et le joint d'étanchéité 17, en empêchant ultérieurement toute dissociation intempestive axiale de l'ajutage 26 en dehors du joint d'étanchéité 17 et de l'orifice 14 de distribution. L'épaulement anti-retour peut être en forme de couronne radiale. La surépaisseur 35 présente un diamètre externe suffisamment important pour réaliser un contact hermétique avec le joint 17 d'étanchéité dès qu'elle arrive au contact de ce joint 17. Après le passage de la surépaisseur 35 au-delà du joint 17, la paroi externe 32 assure le maintien de l'étanchéité au fluide de traitement.

Dans un mode de réalisation, l'extrémité axiale 34 de l'ajutage 26 peut aussi elle-même assurer une étanchéité avec le joint 17.

Dans un autre mode de réalisation, l'extrémité axiale 34 présente un diamètre plus faible que le joint 17, de sorte que l'étanchéité n'est obtenue qu'à partir du moment où la surépaisseur 35 vient au contact du joint 17. Ce dimensionnement a néanmoins l'avantage de faciliter la rupture de l'opercule 31, et l'introduction et le centrage initial de l'ajutage 26 dans l'orifice 14 et la cavité 24.

Dans ce deuxième mode de réalisation, la longueur axiale de l'extrémité axiale 34 de l'ajutage 26 est adaptée (suffisamment faible) pour que le contact hermétique puisse être établi entre la surépaisseur 35 ou la paroi externe 32 de l'ajutage 26, et le joint d'étanchéité 17 lors d'une première course de rapprochement axial et d'introduction dans l'orifice 14, sans que cette extrémité 34 ne vienne au contact de la paroi de la cavité 24 d'actionnement, c'est-à-dire sans ouverture de la valve 10.

Mais la longueur axiale totale de l'ajutage 26, et notamment de la paroi externe 32, est adaptée pour que dans une deuxième course de rapprochement axial au-delà de ladite première course (l'étanchéité étant donc établie), l'extrémité 34 de l'ajutage 26 vienne au contact de paroi de la cavité 24 d'actionnement pour entraîner son déplacement et l'ouverture de la valve 10.

Il est à noter que la présence de l'opercule 31 a aussi pour effet d'obliger l'utilisateur à exercer un effort axial bien aligné avec l'axe de la valve 10 et de valeur suffisante, pour que la manoeuvre d'introduction de l'ajutage 26 dans la valve 10 jusqu'à son actionnement, se déroule correctement, quasi-instantanément et sans risque de fuite.

L'extrémité axiale 34 pénètre alors dans la cavité 24 d'actionnement et permet de repousser la soupape 19 axialement vers l'intérieur jusqu'à ce que le bord d'obturation 20 de la soupape 19 soit décollé du joint d'étanchéité 17. Les formes de l'extrémité axiale 34 de l'ajutage 26 et de la cavité 24 d'étanchéité sont également adaptées pour qu'un passage de fluide de traitement soit possible entre le bord extrême chanfreiné de l'extrémité axiale 34 et le fond 28 de la cavité 24. Dès lors, comme on le voit figure 3, lorsque l'ajutage 26 est introduit à l'intérieur de la valve 10 et que la soupape 19 est repoussée axialement, le fluide de traitement dans la poche 11 peut circuler autour de la soupape 19 dans l'alésage 18, entre le joint 17 et le bord d'obturation 20, entre la cavité 24 et l'extrémité axiale 34, puis peut pénétrer à l'intérieur de l'ajutage 26 pour s'écouler ultérieurement axialement dans le raccord 6 vers l'extérieur de la cartouche 3.

La valve 10 de sortie ainsi formée est donc une valve rappelée élastiquement axialement vers l'extérieur en position d'obturation par le ressort 21, et pouvant être repoussée manuellement axialement vers l'intérieur en vue de son ouverture pour la libération du fluide de traitement et ce, grâce à l'ajutage 26 bi-fonctionnel d'éjection du fluide de traitement et d'actionnement de la valve 10.

Le dispositif 5 de stérilisation par filtration est par exemple un dispositif tel que commercialisé par la société MILLIPORE (Billerica, Massachusetts, USA) sous la dénomination MILLEX®. Un tel dispositif 5 comprend un carter clos hermétique rigide 39 en PVC renfermant un filtre 47 -notamment un microfiltre- formé d'une membrane dont les pores ont une dimension de l'ordre de 0,2µm, et sont aptes à retenir les micro-organismes pathogènes. Ce carter 39 présente une entrée de fluide 38 à connexion tronconique 37 et une sortie de fluide 40. La sortie de fluide 40 forme une connexion tronconique compatible avec son raccord direct à l'extrémité de distribution 41 de la seringue 4. De préférence, la sortie de fluide 40 définit une connexion tronconique femelle à verrouillage par vissage normalisé, notamment de type « Luer-Lok® ».

Le raccord 6 de liaison hermétique amont comprend, à l'opposé de l'ajutage bi-fonctionnel 26, une connexion tronconique 36 conjuguée de la connexion tronconique 37 de l'entrée 38 de fluide du dispositif 5 de stérilisation par filtration. De préférence, ces connexions tronconiques 36, 37 sont conformes aux normes en vigueur, notamment de type Luer®. Ainsi, dans le cas où l'entrée 38 de fluide du dispositif 5 de stérilisation par filtration est de type à connexion mâle, le raccord 6 comprend une connexion tronconique 36 femelle conjuguée. Tel est le cas du mode de réalisation représenté. Il est bien entendu que l'on peut tout aussi bien prévoir que le dispositif 5 de stérilisation par filtration soit doté d'une entrée 38 de fluide à connexion tronconique femelle, et le raccord 6 comprend alors une connexion tronconique mâle conjuguée. La connexion tronconique 36 du raccord 6 et celle 37 de l'entrée de fluide 38 du dispositif 5 de stérilisation par filtration peuvent être soit de type à verrouillage par vissage (notamment du type dit « Luer -Lok®»), soit au contraire de type sans verrouillage, la liaison hermétique entre elles étant obtenue par simple emmanchement relatif.

La seringue d'administration 4 est une seringue jetable standard de volume compris entre 10ml et 100ml, par exemple de 50ml. Cette seringue 4 comprend un piston 42 et un corps de seringue 43 définissant un cylindre 44 dans lequel le piston 42 peut coulisser librement. Le cylindre 44 débouche dans l'extrémité axiale 41 débouchante, dite extrémité de distribution 41, du corps de seringue 43. Dans l'exemple représenté, cette extrémité de distribution 41 forme une connexion tronconique mâle normalisée à verrouillage de type « Luer-Lok®». Cette extrémité de distribution 41 est adaptée pour pouvoir recevoir l'aiguille 7 qui est elle-même dotée d'une connexion tronconique femelle à verrouillage normalisée, de façon traditionnelle. L'aiguille 7 est elle-même emballée dans une poche close 45 et peut être insérée à l'intérieur du corps de seringue 43 dans un évidement de la tige de manoeuvre du piston 42 comme représenté figure 1. Le volume maximum de la cavité de la seringue 4 définie entre l'extrémité de distribution 41 et le piston 42 en position extrême à l'opposé de l'extrémité de distribution 41 est supérieur ou égal à la quantité individuelle de fluide de traitement pouvant être libérée de la cartouche 3 lorsque la valve 10 est ouverte. La cartouche 3 contient en effet uniquement une quantité de fluide de traitement correspondant à une quantité individuelle, destinée à une administration unique, et le cylindre de la seringue peut contenir toute cette quantité individuelle de fluide de traitement. Dans un dispositif selon l'invention, le volume de fluide de traitement contenu dans la cartouche 3 et pouvant être libéré par cette dernière pour une administration unique est ainsi prédéfini à la fabrication. Aucun dosage n'est donc à effectuer par l'utilisateur lors du remplissage de la seringue.

Le bracelet 8 est adapté pour pouvoir être placé autour du poignet du patient traité et ce, dans un double but. Tout d'abord, ce bracelet 8 permet au personnel soignant de savoir immédiatement que le patient a subi un traitement par administration de fluide, notamment de gaz. Le personnel soignant peut donc éviter des erreurs ultérieures de soin et adopter les précautions nécessaires au patient ainsi traité. En effet, par exemple, certaines précautions thérapeutiques doivent être prises avec les patients ayant subi un traitement par administration de gaz afin d'éviter tout accident. De surcroît, le bracelet 8 peut être doté d'une étiquette 46 qui reprend des informations d'identification de la cartouche 3 de fluide de traitement utilisée portées par cette cartouche 3. D'autres étiquettes semblables peuvent aussi être prévues pour être associées au dossier médical du patient. De la sorte, une « traçabilité » parfaite du fluide de traitement est assurée.

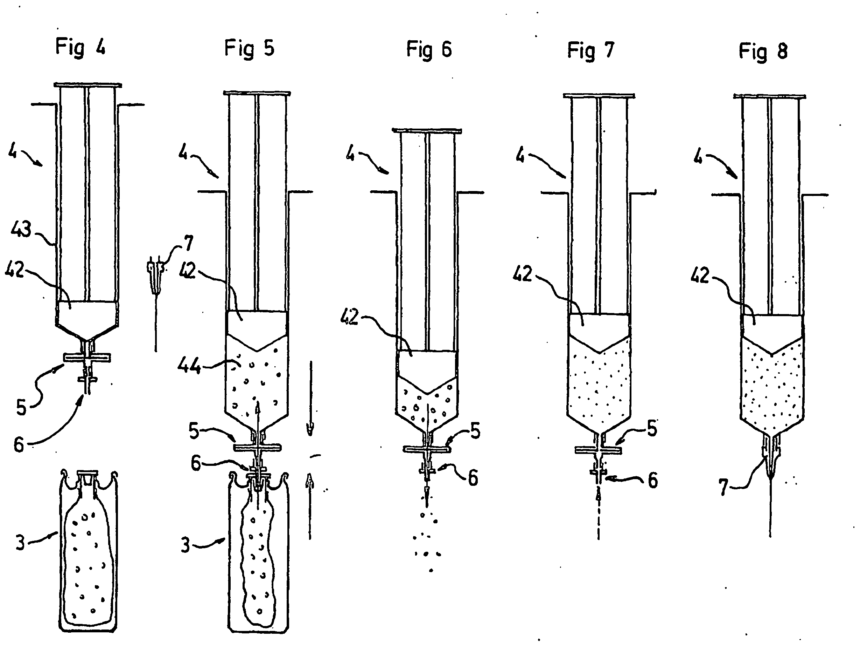

Un exemple de procédé selon l'invention d'utilisation du dispositif selon l'invention est représenté sur les figures 4 à 8, respectivement. Tout d'abord, on ouvre l'enveloppe externe 2 après avoir vérifié que les conditions de stérilisation ont bien été maintenues (l'enveloppe externe 2 peut être dotée de façon traditionnelle d'indicateurs colorés de garantie de l'état stérile). On assemble ensuite axialement la valve 10 de la cartouche 3 à la seringue 4 préalablement dotée du dispositif 5 de stérilisation par filtration et du dispositif 6 de liaison hermétique amont, de façon à réaliser entre eux une communication isolée hermétiquement de l'extérieur.

Il est à noter que la seringue 4 et le dispositif 5 de stérilisation, et de préférence aussi le dispositif 6 de liaison hermétique amont, sont préalablement assemblés les uns aux autres avant l'emballage dans l'enveloppe 2 et la stérilisation à l'oxyde d'éthylène de l'invention. De la sorte, pour réaliser ladite communication hermétique, il suffit de réunir le dispositif 6 de liaison hermétique amont (déjà solidaire du dispositif 5 et de la seringue 4) et la valve 10 de la cartouche 3. L'assemblage préalable de la seringue 4 et du dispositif 5 de stérilisation permet d'éviter tout contact de la cavité interne de la seringue 4 avec l'air extérieur. On garantit ainsi que le contenu de cette cavité est toujours parfaitement stérile.

On rapproche ensuite axialement la seringue 4 et la cartouche 3 de façon à ouvrir la valve 10 et à libérer le fluide de traitement de la cartouche 3 qui s'écoule dans la seringue 4 dont le piston 42 est repoussé spontanément à l'extérieur du corps de seringue 43 par la pression de fluide de traitement ainsi introduite à l'intérieur du cylindre 44. L'introduction de la quantité individuelle de fluide de traitement dans la seringue 4 se produit donc par équilibrage des pressions alors que l'on a rapproché la seringue 4 de la cartouche 3. Le fluide de traitement passant dans le dispositif de stérilisation 5 est stérilisé au fur et à mesure de son introduction dans la cavité de la seringue 4.

On dissocie ensuite la cartouche 3 de la seringue 4. De préférence, on déconnecte le raccord 6 de la valve 10 de la cartouche 3, par exemple en effectuant des mouvements alternatifs d'angulation successifs tout en exerçant une traction axiale, de façon à faire ressortir l'ajutage 26 (et la surépaisseur 35) de l'orifice 14. La présence de l'ajutage 26 non compatible avec l'aiguille 7 empêche tout montage accidentel ultérieur de cette aiguille 7 avant la séparation du dispositif 5 de stérilisation par filtration de la seringue 4.

Dans le cas où le fluide de traitement est un gaz, on peut alors procéder éventuellement à une dilution du gaz par de l'air atmosphérique en éjectant le surplus de gaz hors du cylindre 44 et en aspirant à l'intérieur de ce cylindre 44 une quantité d'air atmosphérique de dilution, via le dispositif 5 de stérilisation par filtration qui stérilise donc aussi cet air de dilution. Ces deux étapes correspondant à la dilution ne sont pas nécessaires, notamment dans le cas où l'on utilise le fluide de traitement à l'état pur. On dissocie ensuite le dispositif 5 de stérilisation par filtration de la seringue 4 et on raccorde l'aiguille 7 rapidement à l'extrémité axiale 41 de la seringue. La seringue ainsi obtenue représentée figure 8 est prête à l'utilisation pour une administration au patient.

Il est à noter qu'après l'étape de remplissage du cylindre de la seringue 4, il reste toujours possible de réinjecter une partie du fluide de traitement dans la cartouche 3 en agissant sur le piston 42, via la communication hermétique ainsi réalisée. Ce faisant, la surépaisseur tronconique 35 de l'ajutage 26 empêche toute dissociation intempestive de la cartouche 3 par rapport au raccord 6. Il est même possible de réinjecter dans la cartouche 3 l'intégralité du fluide de traitement et de réitérer l'opération de remplissage, dans le cas où cette dernière ne se serait pas déroulée de façon considérée comme correcte.

L'ensemble du dispositif selon l'invention après emballage peut être stérilisé à l'oxyde d'éthylène de façon traditionnelle, à faible coût et avec une grande fiabilité. La présence de l'opercule 31 permet d'éviter toute pénétration d'oxyde d'éthylène à l'intérieur de la valve 10 et au contact du fluide de traitement. Le dispositif 1 se présente alors en un ensemble emballé d'un seul tenant, stérile prêt à l'emploi.

L'invention peut faire l'objet de nombreuses variantes de réalisation par rapport au mode de réalisation décrit ci-dessus et représenté sur les figures. Par exemple, la cartouche 3 peut contenir uniquement un gaz de traitement sous pression (sans poche de séparation, tel qu'un gaz fluorocarboné, notamment choisi parmi CF₄, C₂F₆, C₃F₈, ou SF₆, CO₂, N₂, ou de l'air). L'ajutage bi-fonctionnel peut être directement formé par l'entrée du dispositif 5 de stérilisation par filtration. L'opercule 31 peut être réalisé de façon à présenter un renfoncement de guidage de l'ajutage en regard de l'orifice 14. Les différentes liaisons tronconiques peuvent être remplacées en tout ou partie par toute autre forme de liaison étanche au fluide de traitement (assemblage cylindrique collé, assemblage à joint...).

## Revendications

1. Dispositif de préparation extemporanée d'une quantité individuelle de fluide de traitement stérile pouvant être compressible en vue de son administration à un patient, comprenant :
- une seringue (4) d'administration d'une dose de fluide de traitement, comprenant un piston (42) coulissant dans un cylindre (44) doté d'une extrémité axiale (41) débouchante, dite extrémité de distribution (41),
- un réservoir (3) contenant une quantité de fluide de traitement sous pression formé d'une cartouche (3) rigide dotée d'une valve (10) de sortie adaptée pour pouvoir être reliée hermétiquement en communication de fluide avec l'extrémité de distribution (41) de la seringue (4), et pour pouvoir être ouverte en vue de la libération du fluide de traitement sous pression dans le cylindre de la seringue,
**caractérisé en ce que** :
- il comporte un dispositif (5, 6) de raccordement/stérilisation comportant un dispositif (5) de stérilisation par filtration doté d'une entrée (38) de fluide et d'une sortie (40) de fluide, et apte à stériliser le fluide de traitement s'écoulant entre l'entrée (38) et la sortie (40), ce dispositif (5, 6) de raccordement/stérilisation étant adapté pour permettre l'assemblage et le raccordement hermétique de la cartouche (3) et de la seringue (4) en vue du remplissage de la seringue (4), avec le dispositif (5) de stérilisation interposé entre la cartouche (3) et la seringue (4), de telle sorte que la sortie (40) de fluide du dispositif (5) de stérilisation puisse délivrer, dans la seringue (4), du fluide stérile issu de la cartouche (3),
- le réservoir (3) contient une quantité de fluide de traitement sous pression telle que la quantité totale de fluide de traitement pouvant être libérée à la pression atmosphérique par la cartouche (3) lors de l'ouverture de la valve (10) correspond à une quantité individuelle de fluide de traitement pouvant être entièrement contenue dans le cylindre (44) de la seringue (4),
- la valve (10) de sortie est du type rappelé élastiquement axialement vers l'extérieur en position d'obturation et pouvant être repoussée axialement vers l'intérieur en vue de son ouverture pour la libération du fluide de traitement.

2. Dispositif selon la revendication 1, **caractérisé en ce que** le dispositif (5, 6) de raccordement/stérilisation est adapté pour réaliser un assemblage axial de la seringue (4), de ce dispositif (5, 6) de raccordement/stérilisation, et de la valve (10) de la cartouche, et pour permettre le remplissage de la seringue (4), sans fuite de fluide de traitement, par simple rapprochement axial de la seringue (4) et de la cartouche (3).

3. Dispositif selon la revendication 2, **caractérisé en ce que** le dispositif (5, 6) de raccordement/stérilisation comprend une portion (26) de raccordement à la valve (10) de la cartouche (3), et **en ce que** cette portion (26) de raccordement et cette valve (10) sont adaptées pour que :
- dans une première course de rapprochement axial relatif, un raccord hermétique puisse être réalisé entre elles, sans actionnement de la valve (10) de la cartouche (3) qui reste fermée,
- dans une deuxième course de rapprochement axial relatif ultérieur au-delà de la première course, la valve (10) soit ouverte.

4. Dispositif selon la revendication 3, **caractérisé en ce que** :
- la valve (10) de la cartouche (3) est une valve femelle dotée d'un orifice (14) de distribution, d'un joint (17) périphérique d'étanchéité, et d'une cavité (24) d'actionnement adaptée pour recevoir une extrémité (24) d'un ajutage (26) bi-fonctionnel d'actionnement de la valve (10) et d'éjection du fluide de traitement,
- ladite portion (26) de raccordement du dispositif (5, 6) de raccordement/stérilisation forme un ajutage (26) bi-fonctionnel compatible avec la valve (10) femelle de la cartouche (3).

5. Dispositif selon la revendication 4, **caractérisé en ce que** le dispositif (5, 6) de raccordement/stérilisation comprend un dispositif (6) de liaison hermétique amont présentant, d'un côté, ledit ajutage (26) bi-fonctionnel compatible avec la valve (10) femelle de la cartouche (3), et de l'autre côté, une connexion (36) compatible avec l'entrée (38) du dispositif (5) de stérilisation par filtration.

6. Dispositif selon la revendication 5, **caractérisé en ce que** ladite connexion (36) du dispositif (6) de liaison hermétique amont est une liaison tronconique conjuguée d'une liaison tronconique (37) formée par l'entrée du dispositif (5) de stérilisation par filtration.

7. Dispositif selon l'une des revendications 1 à 6, **caractérisé en ce que** la sortie (40) du dispositif (5) de stérilisation par filtration est adaptée pour pouvoir être connectée directement à l'extrémité de distribution (41) de la seringue (4) d'administration.

8. Dispositif selon l'une des revendications 1 à 7, **caractérisé en ce que** la sortie (40) du dispositif (5) de stérilisation par filtration est une liaison tronconique femelle conjuguée d'une liaison tronconique mâle formée par l'extrémité de distribution (1) de la seringue (4) d'administration.

9. Dispositif selon l'une des revendications 1 à 8, **caractérisé en ce que** la cartouche (3) comprend une poche (11) interne souple renfermant ladite quantité individuelle de fluide de traitement sous pression raccordée à la valve (10) de sortie, et à l'extérieur de la poche (11), une quantité de gaz pousseur adaptée à la mise sous pression du fluide de traitement contenu dans la poche (11).

10. Dispositif selon la revendication 9, **caractérisé en ce que** le gaz pousseur est un gaz physiologiquement acceptable.

11. Dispositif selon l'une des revendications 9 ou 10, **caractérisé en ce que** le gaz pousseur est de même nature que le fluide de traitement.

12. Dispositif selon l'une des revendications 9 à 11, **caractérisé en ce que** le fluide de traitement est un gaz de traitement.

13. Dispositif selon l'une des revendications 9 à 12, **caractérisé en ce que** la quantité de gaz pousseur est adaptée pour maintenir dans la poche (11) une surpression de fluide de traitement inférieure à 2000 hPa -notamment de l'ordre de 600 à 1800 hPa-.

14. Dispositif selon l'une des revendications 1 à 13, **caractérisé en ce que** la cartouche (3) comprend un volume de fluide de traitement compris entre 10ml et 100ml -notamment de l'ordre de 40ml-.

15. Dispositif selon la revendication 4 et l'une des revendications 1 à 14, **caractérisé en ce que** la cartouche (3) comprend un opercule (31) de fermeture hermétique de l'orifice (14) de la valve (10) femelle adapté pour empêcher toute pénétration de fluide de stérilisation dans la valve (10) et la cartouche (3), et pour pouvoir être brisé par introduction de l'ajutage (26) bi-fonctionnel dans l'orifice (14) de la valve (10).

16. Dispositif selon l'une des revendications 1 à 15, **caractérisé en ce qu'**il comprend une aiguille (7) stérile apte à être raccordée à l'extrémité de distribution (41) de la seringue (4) pour l'administration du fluide de traitement au patient.

17. Dispositif selon l'une des revendications 1 à 16, **caractérisé en ce qu'**il comprend une étiquette (46) -notamment sous forme de bracelet (8)-destinée à être associée au patient.

18. Dispositif selon l'une des revendications 1 à 17, **caractérisé en ce qu'**il comprend une enveloppe (2) externe d'emballage stérile renfermant l'intégralité des éléments (3, 4, 5, 6, 7, 8) constitutifs du dispositif à l'état stérile.

19. Dispositif selon la revendication 18, **caractérisé en ce que** la seringue (4) et le dispositif (5) de stérilisation se présentent préalablement assemblés à l'état stérile dans l'enveloppe (2).

20. Procédé de préparation extemporanée d'une quantité individuelle de fluide de traitement stérile pouvant être compressible en vue de son administration à un patient, **caractérisé en ce que** :
- on utilise un dispositif (1) selon l'une des revendications 1 à 19,
- on assemble axialement la seringue (4), le dispositif (5, 6) de raccordement/stérilisation, la valve (10) de la cartouche (3), de façon à réaliser entre eux une communication isolée hermétiquement de l'extérieur,
- puis on rapproche axialement la seringue (4) et la cartouche (3) de façon à ouvrir la valve (10) et à introduire dans la seringue (4) une dose prédéterminée de fluide de traitement stérile, par équilibrage des pressions.

21. Procédé selon la revendication 20, **caractérisé en ce qu'**on dilue le fluide de traitement dans la seringue (4).

22. Procédé selon l'une des revendications 20 ou 21, **caractérisé en ce que** la seringue (4) et le dispositif (5) de stérilisation étant préalablement assemblés, on réalise l'assemblage axial uniquement en reliant l'entrée du dispositif de stérilisation à la valve (10) de la cartouche (3).

23. Procédé selon l'une des revendications 20 à 22, **caractérisé en ce qu'**on dissocie ensuite le dispositif (5) de stérilisation par filtration de la seringue (4) à laquelle on raccorde une aiguille (7) d'administration.

## Patentansprüche

1. Vorrichtung zur extemporierten Präparation einer individuellen Menge Flüssigkeit zur sterilen Behandlung, die im Hinblick auf ihre Verabreichung an einen Patienten komprimierbar sein kann, umfassend:
- eine Spritze (4) zur Verabreichung einer Flüssigkeitsdosis zur Behandlung mit einem Kolben (42), der in einem Zylinder (44) gleitet, der mit einem einmündenden axialen Ende (41), einem so genannten Verteilerende (41) versehen ist,
- einen eine Flüssigkeitsmenge zur Behandlung unter Druck enthaltenden Behälter (3), der aus einer steifen Kartusche (3) gebildet wird, die mit einem Austrittsventil (10) versehen ist, das angepasst ist, um hermetisch in Kommunikation mit der Flüssigkeit mit dem Verteilerende (41) der Spritze (4) verbunden zu werden und um im Hinblick auf die Freisetzung der Flüssigkeit zur Behandlung unter Druck in dem Zylinder der Spritze geöffnet zu werden,
**dadurch gekennzeichnet, dass**:
- sie eine Anschluss- / Sterilisationsvorrichtung (5, 6) mit einer Sterilisationsvorrichtung (5) per Filterung umfasst, die mit einem Flüssigkeitseintritt (38) und einem Flüssigkeitsaustritt (40) versehen ist und geeignet ist, die Flüssigkeit zur Behandlung zu sterilisieren, die sich zwischen dem Eintritt (38) und dem Austritt (40) ergießt, wobei diese Anschluss- / Sterilisationsvorrichtung (5, 6) angepasst ist, um den Zusammenbau und den hermetischen Anschluss der Kartusche (3) und der Spritze (4) im Hinblick auf das Befüllen der Spritze (4) zu erlauben, wobei die Sterilisationsvorrichtung (5) derart zwischen der Kartusche (3) und der Spritze (4) zwischengeschaltet ist, dass der Flüssigkeitsaustritt (40) der Sterilisationsvorrichtung (5) in der Spritze (4) aus der Kartusche (3) stammende sterile Flüssigkeit abgeben kann,
- der Behälter (3) eine Menge Flüssigkeit zur Behandlung unter Druck derart enthält, dass die gesamte Menge der Flüssigkeit zur Behandlung, die bei atmosphärischem Druck durch die Kartusche (3) beim Öffnen des Ventils (10) freigesetzt werden kann, einer individuellen Menge Flüssigkeit zur Behandlung entspricht, die vollständig im Zylinder (44) der Spritze (4) enthalten sein kann.
- das Austrittsventil (10) von dem Typ ist, der elastisch axial zur Außenseite in der Verschlussposition zurückgeholt werden kann und axial zur Innenseite im Hinblick auf ihre Öffnung für die Freisetzung der Flüssigkeit zur Behandlung zurückgeschoben werden kann.

2. Vorrichtung gemäß Anspruch 1, **dadurch gekennzeichnet, dass** die Anschluss- / Sterilisationsvorrichtung (5, 6) angepasst ist, um einen axialen Zusammenbau der Spritze (4) dieser Anschluss- / Sterilisationsvorrichtung (5, 6) und des Ventils (10) der Kartusche zu realisieren und um das Befüllen der Spritze (4) ohne Lecken der Flüssigkeit zur Behandlung durch einfaches axiales Annähern der Spritze (4) und der Kartusche (3) zu erlauben.

3. Vorrichtung gemäß Anspruch 2, **dadurch gekennzeichnet, dass** die Anschluss- / Sterilisationsvorrichtung (5, 6) einen Anschlussabschnitt (26) des Ventils (10) der Kartusche (3) umfasst und dass dieser Anschlussabschnitt (26) und dieses Ventil (10) angepasst sind, damit:
- in einem ersten Hub der relativen axialen Annäherung ein hermetischer Anschluss zwischen ihnen ohne Betätigung des Ventils (10) der Kartusche (3) realisiert werden kann, das geschlossen bleibt,
- in einem zweiten Hub der späteren relativen axialen Annäherung über den ersten Hub hinaus, das Ventil (10) offen ist.

4. Vorrichtung gemäß Anspruch 3, **dadurch gekennzeichnet, dass**:
- das Ventil (10) der Kartusche (3) ein weibliches Ventil ist, das mit einer Verteileröffnung (14), einer peripherischen Abdichtungsdichtung (17) und einer Betätigungsvertiefung (24) ausgestattet ist, die angepasst ist, um ein Ende (24) eines bifunktionalen Ansatzes (26) des Ventils (10) und Ausstoßens der Flüssigkeit zur Behandlung aufzunehmen,
- der genannte Anschlussabschnitt (26) der Anschluss- / Sterilisationsvorrichtung (5, 6) einen bifunktionalen Ansatz (26) bildet, der mit dem weiblichen Ventil (10) der Kartusche (3) kompatibel ist.

5. Vorrichtung gemäß Anspruch 4, **dadurch gekennzeichnet, dass** die Anschluss- / Sterilisationsvorrichtung (5, 6) eine obere hermetische Verbindungsvorrichtung (6) umfasst, die auf einer Seite den genannten, mit dem weiblichen Ventil (10) der Kartusche (3) kompatiblen bifunktionalen Ansatz (26) aufweist und auf der anderen Seite eine mit dem Eintritt (38) der Sterilisationsvorrichtung (5) per Filterung kompatible Verbindung (36).

6. Vorrichtung gemäß Anspruch 5, **dadurch gekennzeichnet, dass** die genannte Verbindung (36) der oberen hermetischen Verbindungsvorrichtung (6) eine kegelstumpfartige Bindung ist, die mit einer kegelstumpfartigen Bindung (37) gekoppelt ist, die durch den Eintritt der Sterilisationsvorrichtung (5) per Filterung gebildet wird.

7. Vorrichtung gemäß Anspruch 1 bis 6, **dadurch gekennzeichnet, dass** der Austritt (40) der Sterilisationsvorrichtung (5) per Filterung angepasst ist, um direkt am Verteilerende (41) der Verabreichungsspritze (4) angeschlossen zu werden.

8. Vorrichtung gemäß Anspruch 1 bis 7, **dadurch gekennzeichnet, dass** der Austritt (40) der Sterilisationsvorrichtung (5) per Filterung eine weibliche kegelstumpfartige Bindung ist, die mit einer männlichen kegelstumpfartigen Bindung gekoppelt ist, welche durch das Verteilerende (1) der Spritze (4) zur Verabreichung gebildet wird.

9. Vorrichtung gemäß Anspruch 1 bis 8, **dadurch gekennzeichnet, dass** die Kartusche (3) einen weichen internen Beutel (11) umfasst, der die genannte individuelle Flüssigkeitsmenge zur Behandlung unter Druck umschließt, die an das Austrittsventil (10) angeschlossen ist, und an der Außenseite des Beutels (11) eine Menge Treibgas, die an das Unter-Drucksetzen der in dem Beutel (11) enthaltenen Flüssigkeit zur Behandlung angepasst ist.

10. Vorrichtung gemäß Anspruch 9, **dadurch gekennzeichnet, dass** das Treibgas ein physiologisch akzeptables Gas ist.

11. Vorrichtung gemäß Anspruch 9 oder 10, **dadurch gekennzeichnet, dass** das Treibgas von derselben Art ist wie die Flüssigkeit zur Behandlung.

12. Vorrichtung gemäß Anspruch 9 bis 11, **dadurch gekennzeichnet, dass** die Flüssigkeit zur Behandlung ein Gas zur Behandlung ist.

13. Vorrichtung gemäß Anspruch 9 bis 12, **dadurch gekennzeichnet, dass** die Menge Treibgas angepasst ist, um in einem Beutel (11) einen Überdruck der Flüssigkeit zur Behandlung von weniger als 2.000 hPa - insbesondere in der Größenordnung von 600 bis 1.800 hPa - aufrechtzuerhalten.

14. Vorrichtung gemäß Anspruch 1 bis 13, **dadurch gekennzeichnet, dass** die Kartusche (3) ein zwischen 10 ml und 100 ml - und insbesondere in der Größenordnung von 40 ml - inbegriffenes Flüssigkeitsvolumen zur Behandlung umfasst.

15. Vorrichtung gemäß Anspruch 4 und gemäß Anspruch 1 bis 14, **dadurch gekennzeichnet, dass** die Kartusche (3) ein Innenhütchen (31) zum hermetischen Verschluss der Öffnung (14) des weiblichen Ventils (10) umfasst, das angepasst ist, um jedes Eindringen von Flüssigkeit zur Sterilisation im Ventil (10) und der Kartusche (3) zu verhindern und um durch Einführen des bifunktionalen Ansatzes (26) in die Öffnung (14) des Ventils (10) gebrochen werden zu können.

16. Vorrichtung gemäß Anspruch 1 bis 15, **dadurch gekennzeichnet, dass** sie eine sterile Nadel (7) umfasst, die geeignet ist, am Verteilerende (41) der Spritze (4) zur Verabreichung der Flüssigkeit an den Patienten zur Behandlung angeschlossen zu werden.

17. Vorrichtung gemäß Anspruch 1 bis 16, **dadurch gekennzeichnet, dass** sie ein Etikett (46) - insbesondere in Form eines Armbandes (8) - umfasst, das dazu bestimmt ist, dem Patienten zugeordnet zu werden.

18. Vorrichtung gemäß Anspruch 1 bis 17, **dadurch gekennzeichnet, dass** sie einen externen Umschlag (2) zur sterilen Verpackung umfasst, die alle Elemente (3, 4, 5, 6, 7, 8) umschließt, die die Vorrichtung im sterilen Zustand bilden.

19. Vorrichtung gemäß Anspruch 18, **dadurch gekennzeichnet, dass** die Spritze (4) und die Sterilisationsvorrichtung (5) im sterilen Zustand im Umschlag (2) zuvor zusammengesetzt sind.

20. Verfahren zur extemporierten Präparation einer individuellen Menge Flüssigkeit zur sterilen Behandlung, die im Hinblick auf ihre Verabreichung an einen Patienten komprimierbar sein kann, **dadurch gekennzeichnet, dass**:
- eine Vorrichtung (1) gemäß Anspruch 1 bis 19 verwendet wird;
- die Spritze (4), die Anschluss- / Sterilisationsvorrichtung (5, 6) und das Ventil (10) der Kartusche (3) axial zusammengesetzt werden, so dass zwischen ihnen eine von der Außenwelt hermetisch isolierte Kommunikation realisiert wird,
- dann werden die Spritze (4) und die Kartusche (3) axial aneinander angenähert, so dass das Ventil (10) geöffnet wird und in die Spritze (4) eine vorbestimmte Dosis sterile Flüssigkeit zur Behandlung durch Ausgleich der Drücke eingeführt wird.

21. Verfahren gemäß Anspruch 20, **dadurch gekennzeichnet, dass** die Flüssigkeit zur Behandlung in der Spritze (4) verdünnt wird.

22. Verfahren gemäß Anspruch 20 oder 21, **dadurch gekennzeichnet, dass** der axiale Zusammenbau nur unter Verbindung des Eintritts der Sterilisationsvorrichtung mit dem Ventil (10) der Kartusche (3) realisiert wird, wobei die Spritze (4) und die Sterilisationsvorrichtung (5) zuvor zusammengesetzt werden.

23. Verfahren gemäß Anspruch 20 bis 22, **dadurch gekennzeichnet, dass** anschließend die Sterilisationsvorrichtung (5) per Filterung von der Spritze (4) getrennt wird, an die eine Nadel (7) zur Verabreichung angeschlossen wird.

## Claims

1. A device for the extemporaneous preparation of an individual quantity of sterile treatment fluid that may be compressible, for the purposes of administration to a patient, comprising:
- a syringe (4) for administering a dose of treatment fluid, comprising a piston (42) sliding in a cylinder (44) provided with an open axial end (41), so-called distribution end (41),
- a reservoir (3) containing a quantity of treatment fluid under pressure, formed by a rigid cartridge (3) equipped with an outlet valve (10) capable of being hermetically connected in fluid communication with the distribution end (41) of the syringe (4), and capable of being opened in order to release the treatment fluid under pressure into the cylinder of the syringe,
**characterized in that**:
- it comprises a connection/sterilisation device (5, 6) containing a filtration-type sterilisation device (5) provided with a fluid inlet (38) and a fluid outlet (40), and capable of sterilising the treatment fluid flowing between the inlet (38) and the outlet (40), this connection/sterilisation device (5, 6) being capable of permitting the assembly and hermetic connection of the cartridge (3) and syringe (4) for the purposes of filling the syringe (4), with the sterilisation device (5) interposed between the cartridge (3) and the syringe (4), so that the fluid outlet (40) of the sterilisation device (5) can deliver, into the syringe (4), sterile fluid leaving the cartridge (3),
- the reservoir (3) contains a quantity of treatment fluid under pressure such that the total quantity of treatment fluid that can be released at atmospheric pressure by the cartridge (3) during the opening of the valve (10) corresponds to an individual quantity of treatment fluid that can be contained completely in the cylinder (44) of the syringe (4),
- the outlet valve (10) is of the type that can be axially and elastically displaced towards the exterior in the sealing position and can be axially urged towards the interior in order to open the valve so as to release the treatment fluid.

2. A device according to claim 1, **characterized in that** the connection/ sterilisation device (5, 6) is capable of effecting an axial assembly of the syringe (4), this connection/ sterilisation device (5,6), and the valve (10) of the cartridge, and of permitting the filling of the syringe (4) without loss of treatment fluid by simply axially bringing together the syringe (4) and the cartridge (3).

3. A device according to claim 2, **characterized in that** the connection/sterilisation device (5, 6) comprises a connection portion (26) to the valve (10) of the cartridge (3), and wherein this connection portion (26) and this valve (10) are adapted so that:
- in a first relative axial bringing together of the components, an hermetic connection may be formed between them without actuating the valve (10) of the cartridge (3), which remains closed,
- in a second relative axial bringing together of the components subsequent to the first bringing together, the valve (10) is open.

4. A device according to claim 3, **characterized in that**:
- the valve (10) of the cartridge (3) is a female valve provided with a distribution orifice (14), a peripheral gasket (17), and an actuating cavity (24) capable of receiving an end (24) of a bifunctional delivery tube (26) for actuating the valve (10) and ejecting the treatment fluid,
- the said connecting portion (26) of the connection/sterilisation device (5, 6) forms a bifunctional delivery tube (26) compatible with the female valve (10) of the cartridge (3).

5. A device according to claim 4, **characterized in that** the connection/sterilising device (5, 6) comprises an upstream hermetic connection device (6) having, on one side, the said bifunctional delivery tube (26) compatible with the female valve (10) of the cartridge (3) and, on the other side, a connection (36) compatible with the inlet (38) of the filtration-type sterilisation device (5) .

6. A device according to claim 5, **characterized in that** the said connection (36) of the upstream hermetic connection device (6) is a truncated conical connection paired with a truncated conical connection (37) formed by the inlet of the filtration-type sterilisation device (5).

7. A device according to one of claims 1 to 6, **characterized in that** the outlet (40) of the filtration-type sterilisation device (5) is capable of being able to be connected directly to the axial end (41) of the administration syringe (4).

8. A device according to one of claims 1 to 7, **characterized in that** the outlet (40) of the filtration-type sterilisation device (5) is a female truncated conical connection paired with a male truncated conical connection formed by the axial end (1) of the administration syringe (4).

9. A device according to one of claims 1 to 8, **characterized in that** the cartridge (3) comprises a flexible internal pocket (11) containing the said individual quantity of treatment fluid under pressure connected to the outlet valve (10) and to the exterior of the pocket (11), a quantity of thrust gas capable of pressurising the treatment fluid contained in the pocket (11).

10. A device according to claim 9, **characterized in that** the thrust gas is a physiologically acceptable gas.

11. A device according to one of claims 9 or 10, **characterized in that** the thrust gas is of the same type as the treatment fluid.

12. A device according to one of claims 9 to 11, **characterized in that** the treatment fluid is a treatment gas.

13. A device according to one of claims 9 to 12, **characterized in that** the quantity of thrust gas is capable of maintaining in the pocket (11) an excess pressure of treatment fluid of less than 2000 hPa, in particular of the order of 600 to 1800 hPa.

14. A device according to one of claims 1 to 13, **characterized in that** the cartridge (3) comprises a volume of treatment fluid between 10 ml and 100 ml, in particular of the order of 40 ml.

15. A device according to claim 4 and one of claims 1 to 14, **characterized in that** the cartridge (3) comprises a cap (31) for the hermetic closure of the orifice (14) of the female valve (10) adapted to prevent any penetration of sterilisation fluid into the valve (10) and the cartridge (3), and that can be broken by introduction of the bifunctional delivery tube (26) into the orifice (14) of the valve (10).

16. A device according to one of claims 1 to 15, **characterized in that** it comprises a sterile needle (7) capable of being connected to the end (41) of the syringe (4) for the administration of the treatment fluid to the patient.

17. A device according to one of claims 1 to 16, **characterized in that** it comprises a label (46), in particular in the form of a bracelet (8), designed to be associated with the patient.

18. A device according to one of claims 1 to 17, **characterized in that** it comprises a sterile external packaging (2) containing all the constituent elements (3, 4, 5, 6, 7, 8) of the device in the sterile state.

19. A device according to claim 18, **characterized in that** the syringe (4) and the sterilisation device (5) are assembled beforehand in the sterile state in the packaging (2).

20. A method for the extemporaneous preparation of an individual quantity of sterile treatment fluid that may be compressible, for the purposes of its administration to a patient, **characterized in that**:
- a device (1) according to one of claims 1 to 19 is used,
- the syringe (4), the connection/sterilisation device (5, 6) and the valve (10) of the cartridge (3) are assembled axially so as to form between them a communication that is hermetically sealed from the surroundings,
- the syringe (4) and the cartridge (3) are then brought together axially so as to open the valve (10) and introduce into the syringe (4) a predetermined dose of sterile treatment fluid, by equilibration of the pressures.

21. A method according to claim 20, **characterized in that** the treatment fluid is diluted in the syringe (4).

22. A method according to one of claims 20 or 21, **characterized in that** the syringe (4) and the sterilisation device (5) are assembled beforehand and the axial assembly is obtained only by connecting the inlet of the sterilisation device to the valve (10) of the cartridge (3).

23. A method according to one of claims 20 to 22, **characterized in that** the filtration-type sterilisation device (5) is next disconnected from the syringe (4), to which an administration needle (7) is then connected.
